(19) Europäisches Patentamt
European Patent Office
Office européen des brevets

(11) EP 3 646 785 A1

(12) EUROPEAN PATENT APPLICATION

(43) Date of publication:
**06.05.2020 Bulletin 2020/19**

(21) Application number: **18203207.8**

(22) Date of filing: **29.10.2018**

(51) Int Cl.:
***A61B 5/05*** *(2006.01)* ***A61B 5/00*** *(2006.01)*
***A61B 5/053*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Minchion SA**
**1227 Carouge GE (CH)**

(72) Inventor: **Canepa, Stefano Nicolò**
**1227 Carouge (CH)**

(74) Representative: **Serravalle, Marco et al**
**Marco Serravalle S.L.**
**Carretera Playa San Juan Residence Marazul**
**Bungalow A112**
**38678 Adeje (ES)**

# (54) DEVICE FOR RECOGNITION OF BIOLOGICAL ALTERATION IN HUMAN TISSUES

(57) The present invention is directed to a device for recognition of biological alteration in human tissues using electromagnetic waves in the microwave range, the device comprising: a transmitter device (100) comprising at least one transmitting antenna (101), a transmitter (102), and a power supply (103); a receiving device (200) comprising at least one receiving antenna (201), a receiver (202), a pre-processing module (204), and a power supply (203); and a data processing device (300) comprising a microprocessor (301) and a display (302); wherein the transmitter device (100) and the receiving device (200) are configured to operate at a frequency comprised between 2.0 GHz and 3.0 GHz.

In a preferred embodiment, the operating frequency is comprised between 2.3 GHz and 2.5 GHz, and the device is suitable for the detection of a cancer in the human body, for example for the screening of prostate cancer, colorectal cancer, breast cancer, thyroid cancer.

The device according to the invention is capable of high sensitivity and accuracy of results and can detect not only the presence, but also the position of a cancer.



Printed by Jouve, 75001 PARIS (FR)

## Description

### Field of the invention

**.** The present invention is directed to a device for recognition of biological alteration in tissues, in particular it relates to a device making use of electromagnetic waves with an operating frequency considerably higher than described in the prior art and comprised between 2.0 and 3.0 GHz.

### Background of the invention

**.** The use of radio frequencies for recognition of biological alteration in tissues has been disclosed in several patent applications. WO 01/07909 (Vedruccio) discloses an apparatus comprising a coherent transceiver probe which generates radio frequencies at three different wavelengths (436, 872 and 1308 MHz) and a spectrum analyzer. The probe is manually swept along the body of the patient and the decrease or disappearance of one of the three peaks is considered as an indicator of a type of alteration. Because of this, the three different wavelengths are considered essential. The apparatus, commercially known as Trimprob, was used by many doctors, but considered scarcely reliable since the results are strongly dependent on the operator.

**.** EP 2 364 647 (Centro Studi e Ricerche Sant' Angela srl) discloses an apparatus for the detection of anomalies in biological tissues which tries to overcome the limits of the prior art. In particular, the apparatus addresses the problem of the lack of reliability by keeping the distance between transmitter and antenna constant. The apparatus of '647 tried also to overcome the dependency on the operator by moving the antenna automatically. The preferred frequency range is between 400 and 1000 MHz.

**.** EP 2 465 428 (Medielma srl) is also addressed to the identical problem and tries to solve it by increasing the number of receiving antennas so that one of the receiving antennas is always connected to the transmitter and, consequently, the apparatus is capable of detecting the anomalies. The apparatus operates at a frequency lower than 700 MHz. In fact, the patent considers very important to irradiate the tissue in "near-filed conditions". According to the patent, such condition can only be reached if the wavelength is lower than 700 MHz.

. EP 3 257 439 (Medielma srl) discloses an evolution of the apparatus of '428, wherein the antenna radiating element is rotated of a predetermined angle both in clockwise and counter clockwise direction by means of a servo mechanism. Also in this case, the attempt is to reduce human activity and define a standard method for performing the analysis.

**.** The results of the apparatuses according to the prior art, although useful, are considered as a preliminary screening, since the last word about the presence of a cancer is always left to other exams, e.g. NMR analysis colonoscopy, biopsy, etc.. However, the above exams are expensive and invasive. Thus, it would be highly desirable to be able to reduce the number of patients undergoing the above invasive exams. The present invention represents a very important step in this direction and provides an analytical tool with a reliability which is comparable to the more expensive imaging examinations.

**.** The present invention is directed to a device for recognition of biological alteration in human tissues, more specifically for the detection of a cancer in some organs of the human body, without necessarily go for an imaging examination and it results in a diagnosis which is accurate, not only in terms of the correct assessment whether a cancer is present, but also about the location of the cancer.

### Summary of the invention

**.** The present invention is directed to a device recognition of biological alteration in human tissues, which device comprises a microwave transmitter, a microwave receiver, and a data processing unit and wherein the operating frequency of the microwave transmitter and receiver is comprised between 2.0 and 3.0 GHz. The use of a frequency within this range allows a very reliable diagnosis and a localization of the cancer.

### Brief description of the drawings

**.** Fig. 1 is a block diagram of an embodiment of the device according to the invention.
**.** Figure 2 is a block diagram of another preferred embodiment of the invention.

## Detailed description of the invention

. The invention is directed to a device for the detection of tissue anomalies comprising: a transmitter device comprising at least one transmitting antenna, a transmitter, and a power supply; a receiving device comprising at least one receiving antenna, a receiver, a pre-processing module, and a power supply; and a data processing device comprising a microprocessor, a display and a power supply; wherein the transmitter device and the receiving device are configured to operate at a frequency comprised between 2.0 GHz and 3.0 GHz.

. The block diagram of Fig.1 shows a transmitter device 100 comprising an antenna 101, a transmitter 102, and a power supply 103; a receiver device 200, comprising four receiving antennas 201, a receiver 202, a pre-processing module 204, and a power supply 203; and a data processing device 300 comprising a microprocessor 301, a display 302 and a power supply 303.

. Fig.2 represents another preferred embodiment of the invention wherein the transmitter device 100 further comprises a Bluetooth transmitter/receiver 106, a microprocessor 104 and a display 105. The microprocessor 301 is also connected to a Bluetooth transmitter 306. In this way, the information is transferred through Bluetooth devices 106-306 from microprocessor 301 to microprocessor 104, which allows visualization of the analytical results on the display 105. In this embodiment, the operator can see directly on transmitter 100 the result of the analysis, without any need to look at display 302.

. In one embodiment, the transmitter device comprises a single antenna, which is optionally a resonant cavity dipole antenna, or a directional antenna to be used in case of small organs, and a quartz oscillator to fix the specific emitted radio-frequency.

. The transmitter is preferably structured to radiate a narrowband incident microwave frequency signal to irradiate a patient tissue. To the purpose of the present application, narrowband signal means a signal with a bandwidth Bw which is small enough to use the assumption that a response as a result of the interaction with the body can be considered constant within the bandwidth Bw, provided that 1/Bw is below the relaxation times of the irradiated patient biological tissues (typical Bw>IKHz). The frequency radiated by the transmitter device is comprised in the range from 2.0 GHz to 3.0 GHz, for example from 2.2 GHz to 2.7 GHz, more preferably from 2.3 GHz to 2.5 GHz, most preferably around about 2.4 GHZ, which means from 2.35 GHz to 2.45 GHz.

. The receiver device comprises a power supply, which preferably comprises rechargeable batteries. The receiver also comprises a pre-processing module. The pre-processing module preferably comprises a filter used to clean the signal excluding any other frequency different from the emitted frequency in order to eliminate electromagnetic noise of the environment. Signals from the system of receiving antennas are digitalized through the receiver and sent to the pre-processing module. The signal thus obtained is subsequently sent to a data processing unit, such as a laptop computer or an equivalent console, which through a processing algorithm provides parameters representing measures of the backscattered wave-field in term of different characteristics such as attenuation, polarization, resonances and interferences. Most preferably, the data processing unit is configured to run algorithms which determine the presence of nulls or minima (i.e. values below a threshold) in the received signal and associated with the presence of tissue anomalies, more specifically cancers. The data processing unit also comprises a display and a power supply, which preferably comprises rechargeable batteries.

. The size of the receiver is compact. It is important to note that a receiver for the frequency of 2.4 GHz is small, since the size of an antenna is related to the wavelength $\lambda$ of the radiation (2.4 GHz, $\lambda$= 12 cm). If more than one receiving antenna is used, the length of the receiver is preferably less than 0.80 m, more preferably less than 0.60 m even more preferably less than 0.50 m.

. Although multiple antennas can be present, it is also possible to have a single antenna in the receiving device. The use of a single antenna allows a considerable reduction of size of the receiver device. A receiver comprising a single receiving antenna has preferably a length which is less than 0.40 m, more preferably less than 0.30 m.

. In any case, the width of the receiver is preferably less than 0.30 m and the thickness less than 0.030 m. This reduced size and thickness of the receiver allows it to be placed on the medical couch under the patient in the position corresponding to the organ to be examined.

. In view of the reduced size of both the transmitter and the receiver, the device according to the invention can be easily transported to a patient bed or in a different location without any problem. In fact, the entire device can be placed in a bag, which allows easy transportation. The overall weight of the device according to the invention is very limited, e.g. lower than 30 kg, preferably lower than 25kg, more preferably less than 20 kg.

. The device according to the invention can be used in any room and on any available medical couch and does not require a dedicated medical couch and room.

. For this purpose, the device of the present invention is preferably provided with rechargeable batteries which allow operation of the device without the need of external power supply.

. Another advantage of the device of the present invention is the capability of detecting the position of the cancer. In the case of prostate cancer, the device according to the invention is capable of determining whether the cancer is located on the left or on the right lobe. To obtain this information, to date it was necessary to perform an NMR analysis. Analogously, for colorectal cancer, the device according to the invention allows a diagnosis of the intestinal tract where the cancer is present.

. The method of use of the device according to the invention is simple. The receiver is placed under the patient in correspondence to the organ to be checked. Then, the transmitter is turned on and placed in contact with the body as close as possible to the organ to be examined. The transmitter probe is tilted and the system generates a graphical interpretation.

**Experimental part**

**Prostate examination**

. 155 urologic patients were evaluated by multiparametric NMR, and by the device according to the invention (2.4 GHz). The results are confronted with the results of a prostate transrectal eco guided biopsy, which is considered the gold standard in prostate cancer diagnosis.

. For each methodology, two different analyses were performed: a first analysis on the entire prostate (Table 1), and a second analysis to assess the laterality of a possible prostate cancer (Table 2).

Table 1

| | 2.4 GHz | NMR |
|---|---|---|
| True Positive (TP) | 19 | 15 |
| False Positive (FN) | 7 | 6 |
| True Negative (TN) | 129 | 130 |
| False Negative (FN) | 0 | 4 |

Table 2

| | 2.4 GHz | NMR |
|---|---|---|
| True Positive (TP) | 22 | 19 |
| False Positive (FN) | 10 | 8 |
| True Negative (TN) | 275 | 277 |
| False Negative (FN) | 3 | 6 |

. The results on table 1 show that the device according to the invention did not produce any false negative and a number of false positive equal to the number obtained by NMR. It is important to note that false negatives are the most dangerous cases, since when a false negative occurs, a person which is diagnosed sane, is in fact affected by a cancer. Tables 3 and 4 report a statistical evaluation of the data of table 1 and 2 respectively by calculating sensitivity, specificity, negative predictive value (NPV), positive predictive value (PPV), and accuracy, wherein:

.

$$\text{Sensitivity} = TP/(TP+FN)\cdot 100$$

.

$$\text{Specificity} = TN/(TN+FP)\cdot 100$$

.

$$PPV = TP/(TP+FP)\cdot 100$$

.

$$NPV = TN/(TN+FN)\cdot 100$$

.

$$Accuracy = (TP+TN)/(TP+FP+TN+FN)\cdot 100$$

Table 3 Statistical analysis of the results of Table 1

| | Sensitivity | Specificity | PPV | NPV | Accuracy |
|---|---|---|---|---|---|
| 2.4 GHz | 100 | 94.85 | 73.08 | 100 | 95.48 |
| NMR | 78.95 | 95.59 | 71.43 | 97.01 | 93.55 |

Table 4 Statistical analysis of the results of Table 2

| | Sensitivity | Specificity | PPV | NPV | Accuracy |
|---|---|---|---|---|---|
| 2.4 GHz | 88.00 | 96.49 | 68.75 | 98.92 | 95.81 |
| NMR | 76.00 | 97.19 | 70.37 | 97.88 | 95.45 |

**.** From the data of tables 3 and 4 it is evident that the device of the present invention is highly reliable and allows a screening of patients to detect the presence of a prostate cancer in an easy and economical manner. The device is also suitable for recognition of alteration of other organs, for example for the screening of colorectal cancer, breast cancer, thyroid cancer.

## Claims

**1.** Device for the detection of tissue anomalies comprising:

a. a transmitter device (100) comprising at least one transmitting antenna (101), a transmitter (102), and a power supply (103);
b. a receiving device (200) comprising at least one receiving antenna (201), a receiver (202), a pre-processing module (204), and a power supply (203); and
c. a data processing device (300) comprising a microprocessor (301) and a display (302);

wherein the transmitter device (100) and the receiving device (200) are configured to operate at a frequency comprised between 2.0 GHz and 3.0 GHz.

**2.** Device according to claim 1, wherein the operating frequency is comprised between 2.1 GHz and 2.7 GHz

**3.** Device according to claim 1, wherein the operating frequency is comprised between 2.3 GHz and 2.5 GHz.

**4.** Device according to claims 1-3, wherein the transmitter is structured to radiate a narrowband incident radiofrequency signal.

**5.** Device according to claims 1-4, wherein the transmitter device comprises a single antenna, and a quartz oscillator to fix the specific emitted radio-frequency.

**6.** Device according to claims 1-5, wherein the transmitting antenna (101) is a resonant cavity dipole antenna, or a directional antenna.

**7.** Device according to claims 1-6, wherein the power supply (103, 203, 303) comprises a rechargeable battery.

**8.** Device according to claims 1-7, wherein the transmitter device (100) further comprises a Bluetooth transmitter/receiver (106), a microprocessor (104) and a display (105); the microprocessor (301) is also connected to a Bluetooth transmitter (306); wherein the microprocessor (301) is capable of communicating to the microprocessor (104) through Bluetooth devices (306;106), and the information is visualized on display (105).

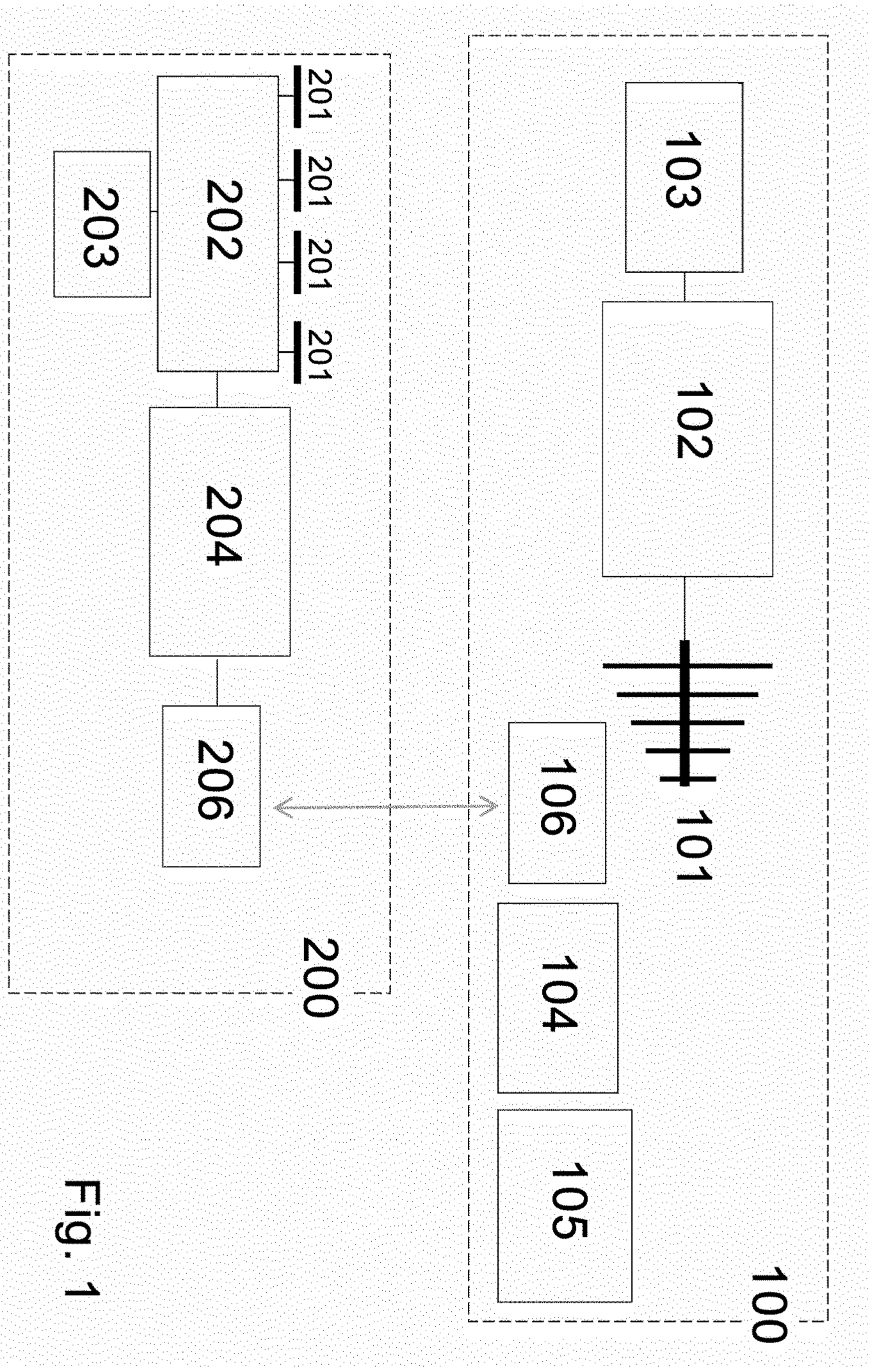


Fig. 1

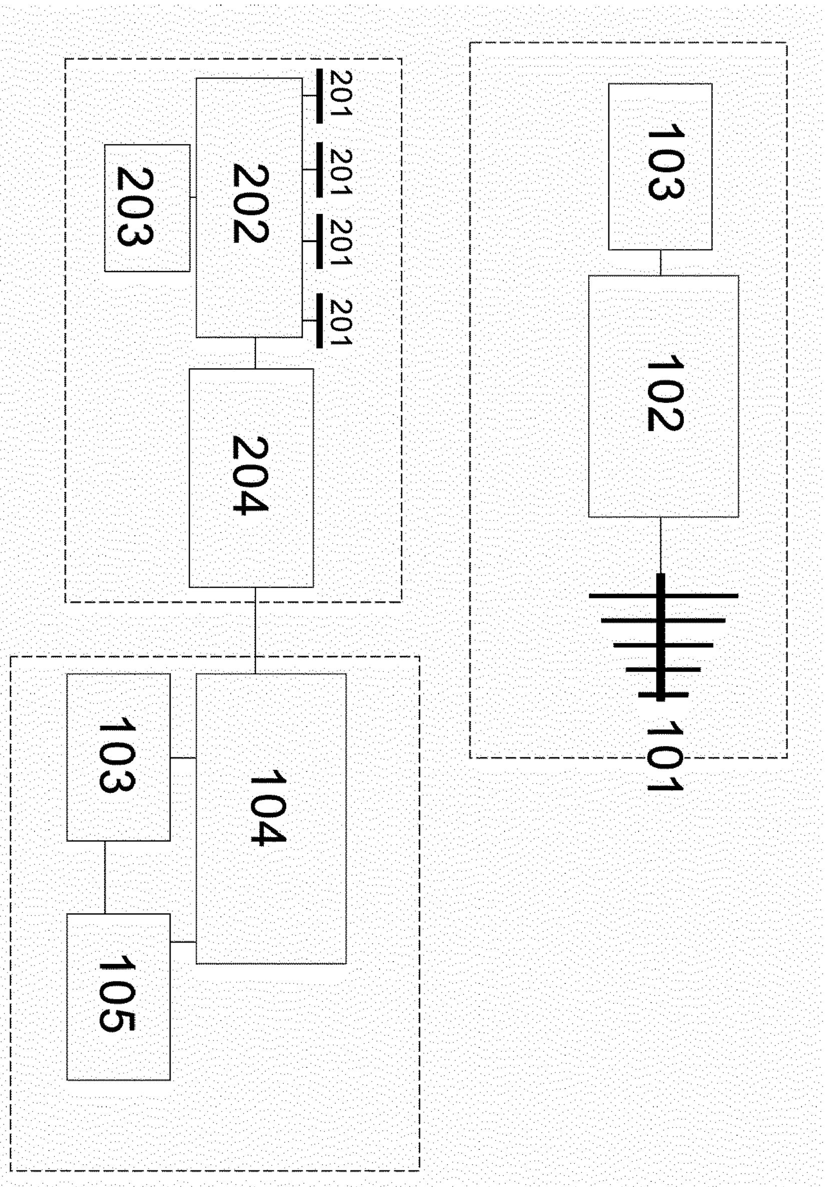


Fig. 2

Europäisches Patentamt
European Patent Office
Office européen des brevets

# EUROPEAN SEARCH REPORT

Application Number
EP 18 20 3207

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/007149 A1 (TING SSU-HAN [TW]) 12 January 2017 (2017-01-12) * abstract; figures 1,2 * * paragraphs [0006] - [0009], [0013] - [0022] * ----- | 1-8 | INV. A61B5/05 A61B5/00 A61B5/053 |
| X,D | EP 3 257 439 A1 (MEDIELMA S R L [IT]) 20 December 2017 (2017-12-20) * abstract; figures 1-4 * * paragraphs [0009] - [0064] * ----- | 1-8 | |
| A | US 9 504 404 B1 (SHAO WENYI [US] ET AL) 29 November 2016 (2016-11-29) * column 1, line 55 - column 2, line 67 * ----- | 1-8 | |
| A,D | EP 2 465 428 A1 (MEDIELMA S R L [IT]) 20 June 2012 (2012-06-20) * abstract; figures 1-12 * * paragraphs [0020] - [0050] * ----- | 1-8 | |
| | | | TECHNICAL FIELDS SEARCHED (IPC) |
| | | | A61B |

The present search report has been drawn up for all claims

| Place of search | Date of completion of the search | Examiner |
|---|---|---|
| The Hague | 21 March 2019 | Carta, Riccardo |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 20 3207

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2019

| Patent document cited in search report | Publication date | Patent family member(s) | Publication date |
|---|---|---|---|
| US 2017007149 A1 | 12-01-2017 | CN 106333645 A | 18-01-2017 |
| | | TW 201701828 A | 16-01-2017 |
| | | US 2017007149 A1 | 12-01-2017 |
| EP 3257439 A1 | 20-12-2017 | AR 108715 A1 | 19-09-2018 |
| | | CN 109310359 A | 05-02-2019 |
| | | EP 3257439 A1 | 20-12-2017 |
| | | SG 11201809919V A | 28-12-2018 |
| | | UY 37291 A | 02-01-2018 |
| | | WO 2017215995 A1 | 21-12-2017 |
| US 9504404 B1 | 29-11-2016 | NONE | |
| EP 2465428 A1 | 20-06-2012 | EP 2465428 A1 | 20-06-2012 |
| | | ES 2632341 T3 | 12-09-2017 |
| | | LT 2465428 T | 25-07-2017 |
| | | MA 34766 B1 | 03-12-2013 |
| | | PL 2465428 T3 | 30-11-2017 |
| | | PT 2465428 T | 17-07-2017 |
| | | WO 2012080300 A1 | 21-06-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0107909 A
- EP 2364647 A
- EP 2465428 A
- EP 3257439 A